# EUROPEAN PATENT APPLICATION

(11) **EP 4 385 630 A1**
(43) Date of publication of application: **19.06.2024**
(21) Application number: 22930656.8
(22) Date of filing: 14.12.2022
(51) Int. Cl.: B06B 1/06, A61B 17/12

(54) **ULTRASONIC CONTROL DEVICE AND ULTRASONIC CONTROL TESTING SYSTEM**

(30) Priority: 08.03.2022 CN 202210222728
(71) Applicant: Jilin University, Changchun, Jilin 130012 (CN)
(72) Inventor: CUI, Shusen, Changchun, Jilin 130012 (CN); LIU, Xilin, Changchun, Jilin 130012 (CN)
(74) Representative: Zaboliene, Reda
(86) International application number: PCT/CN2022/139063
(87) International publication number: WO 2023/169036

(57) **Abstract**

The present disclosure relates to the field of ultrasonic deformation material control. Disclosed are an ultrasonic control device and an ultrasonic control testing system, applied to control or tests of deformation of a material in a human body or an animal body caused by ultrasonic waves. The ultrasonic control device comprises a fixed platform, movable supports, ultrasonic transmitters, and a controller. Due to the safety of ultrasonic waves, the present disclosure can be applied to aspects such as scientific research and treatment of biomedicine.

## Description

### Technical Field

The present disclosure relates to the field of ultrasonic deformation material control, and more particularly, to an ultrasonic control device and a system with controllable direction or energy density, applied to control or tests of deformation of a material in a human body or an animal body caused by ultrasonic waves.

### Background Art

In the treatment or scientific research work for human or living animals, it is often necessary to compress the vessels or nerves of the lesions, resulting in ischemia. The traditional method adopts acute compression for blood vessels or nerves by the surgery. Acute ischemia, however, causes rapid necrosis of local tissues, thereby releasing a large number of fragmented organelles and chromatin fragments to overflow, causing an inflammatory response in surrounding tissues.

The currently available method of chronic ischemia includes surgically implanting an extrusion ring of water-swellable material. When the extrusion ring is implanted in the body, it expands by slowly absorbing body fluid, thereby forming chronic compression on blood vessels. A problem with this approach, however, is that the speed of the compression process is not controllable. Because the body fluid secretion rate changes from site to site and from individual to individual, the swelling rate of the water-swellable material varies. In some sites where the fluid secretion is relatively rapid, the water-swellable material will rapidly swell, resulting in excessive compression and inflammatory response of the surrounding tissues.

At present, there are some ultrasonic-responsive polymer materials, such as polystyrene, polylactide and acrylonitrile based composite materials, of which molding preparation adopts high-pressure molding technology, and a large amount of residual stresses is left in the microstructure. By ultrasonically exciting the oscillations, the residual stresses in the microstructure can be released, resulting in deformation of the material. Since the residual stress release of such materials is directional, high energy density ultrasound is required. However, the traditional ultrasonic oscillation has no directionality, and the energy cannot be concentrated. Thus, the practical application of such materials is scarce, only staying in the laboratory stage.

### Summary of the Invention

In view of the deficiencies of the prior art, it is an object of the present disclosure to provide an ultrasonic control device and a system with controllable direction or energy density to address deformation control of ultrasonic-responsive polymer materials, particularly ultrasonic-responsive materials implanted in the human body or experimental animal body.

In order to achieve the purpose above, the present disclosure provides the following technical solutions.

An ultrasonic control device is characterized in that the ultrasonic control device includes:
a fixed platform configured for placing an object to which ultrasonic waves are to be applied;
a movable support slidably connected to the fixed platform;
an ultrasonic transmitter fixed to the movable support and configured for applying the ultrasonic waves to the object placed on the fixed platform; and
a controller configured for controlling the frequency, energy and vibration direction of the ultrasonic waves emitted by the ultrasonic transmitter.

Optionally, the movable support includes a first movable support and a second movable support which are symmetrically and slidably connected to both sides of the fixed platform, respectively.

Optionally, the ultrasonic transmitter includes a first ultrasonic transmitter fixed to the first movable support and a second ultrasonic transmitter fixed to the second movable support. Optionally, the first ultrasonic transmitter and the second ultrasonic transmitter are disposed orthogonally to each other.

Optionally, the ultrasonic transmitter includes a base, a piezoelectric ceramic plate, an ultrasonic concentrator, a housing and an end cap;
wherein the piezoelectric ceramic plate is sandwiched between the base and the ultrasonic concentrator; and
the housing is fixedly connected to the end cap to enclose the base and the piezoelectric ceramic plate.

Optionally, the ultrasonic control device further includes a cable hole disposed in a central region of the end cap and the base.

Optionally, the ultrasonic concentrator has a front end which is an arc-shaped indent surface for focusing the energy of the ultrasonic waves.

Optionally, the movable support is connected to the fixed platform via a slide groove.

The present disclosure also provides the following technical solutions.

An ultrasonic control system with controllable direction or energy density includes an ultrasonic control device as described above, and further includes a deformable structure, which can be placed in a human or animal body, and which is made of an ultrasonic-responsive material and capable of releasing residual stresses under ultrasound excitation to cause deformation thereof.

According to the above-mentioned technical solution, the present disclosure provides an ultrasonic control device and a system with controllable direction or energy density, wherein the ultrasonic control device may include two ultrasonic transmitters which are orthogonal and perpendicular to each other, and the frequency or power thereof can be independently controlled by the controller. By adjusting the frequency or power of the ultrasonic transmitters, the ultrasonic waves with controllable direction or energy density may be generated in the human body or animal body, so as to realize deformation control of an ultrasonic deformation material pre-implanted in the human body or animal body, thereby satisfying a controllable chronic ischemia treatment or modeling method. Herein, the movable support is mounted on the fixed platform for mounting and fixing the ultrasonic transmitter, and can move in parallel along the fixed platform for adjusting the position of the ultrasonic transducer. Two of the ultrasonic transducers are arranged orthogonal to each other and controlled by the same controller. The controller generates two paths of high-frequency alternating voltages and applies same to both sides of the piezoelectric ceramic plate, and adopts the piezoelectric effect of the piezoelectric ceramic to generate ultrasonic vibration. One end of the ultrasonic concentrator is connected to the piezoelectric ceramic plate, and the other end is an arc-shaped indent surface. The ultrasonic vibration is transmitted from the piezoelectric ceramic to the arc-shaped end, and converted into the ultrasonic waves. The ultrasonic waves are focused at a position 10 cm away from the end surface via the arc-shaped indent surface. During operation, the controller generates two paths of high-frequency voltages with the same frequency and phase or with an adjustable voltage amplitude, and can enable two orthogonally arranged ultrasonic transmitters to generate ultrasonic waves with the same frequency, different phases and controllable energy. The phase difference can be adjusted, and the same is focused to a position at a distance of 10 cm by the ultrasonic concentrator. By adjusting the voltage amplitude, the amplitude of the generated ultrasonic vibration can be controlled so as to control the energy of the ultrasonic wave. By adjusting the phase difference of the two high-frequency voltages, the direction-controllable ultrasonic vibration can be generated at the position where the ultrasonic waves are focused, thereby inducing the local micro-pore rupture of the material, and realizing the deformation of the ultrasonic-responsive material.

The present disclosure has the following beneficial effects.

The present disclosure enables precise control of the direction of energy or vibration of ultrasonic waves within the human or animal body and control of various types of ultrasonic-responsive materials by focusing the ultrasonic waves on a fixed region by the ultrasonic concentrator. By adjusting the amplitude of the output voltage by the controller, the energy of the ultrasonic waves can be controlled so as to maintain control of the ultrasonic-responsive material within a biologically safe energy range. By adjusting the phase difference of the two ultrasonic transmitters by the controller, the direction of the vibration at the focus can be controlled, facilitating the driving of the ultrasonic-responsive material for optimum effects.

### Brief Description of the Drawings

Fig. 1 is an overall schematic structural diagram of an embodiment of the present disclosure.
Fig. 2 is a schematic cross-sectional view of the structure of an ultrasonic transmitter according to an embodiment of the present disclosure;
Fig. 3 is a schematic diagram of the design of an ultrasonic concentrator of according to an embodiment of the present disclosure.
Fig. 4 is a schematic diagram of the operation of the ultrasonic vibration direction control of the present disclosure according to an embodiment of the present disclosure.

In the drawings, 1-fixed platform, 2-movable support, 3-ultrasonic transmitter, 4-human body or experimental animal, 11-base, 12-piezoelectric ceramic plate, 13-ultrasonic concentrator, 14-housing, 15-end cap, 16-front-end fixing screw, 17-back-end fixing screw.

### Detailed Description

Hereinafter, embodiments of the present disclosure will be described with reference to the accompanying drawings. It should be understood, however, that the description is intended for purposes of illustration only, and is not intended to limit the scope of the present disclosure. Further, in the following description, descriptions of well-known structures and techniques are omitted to avoid unnecessarily obscuring the concepts of the present disclosure.

Various structure diagrams according to embodiments of the present disclosure are shown in the accompanying drawings. The figures are not drawn to scale, with some details exaggerated and possibly omitted for clarity of presentation. The shapes of the various regions and layers, and the relative sizes and positional relationships between them shown in the drawings are merely exemplary, and in practice may deviate due to manufacturing tolerances or technical limitations. A person skilled in the art may additionally design regions/layers having different shapes, sizes, relative positions according to actual needs.

In the context of this disclosure, when a layer/element is referred to as being "on" another layer/element, the layer/element may be directly on the other layer/element or intervening layers/elements may be present between them. In addition, if a layer/element is "above" another layer/element in one orientation, the layer/element may be "below" the other layer/element when the orientation is reversed.

The present disclosure may be presented in various forms, some examples of which are described below.

The following is combined with the accompanying drawings to explain the embodiments of this disclosure.

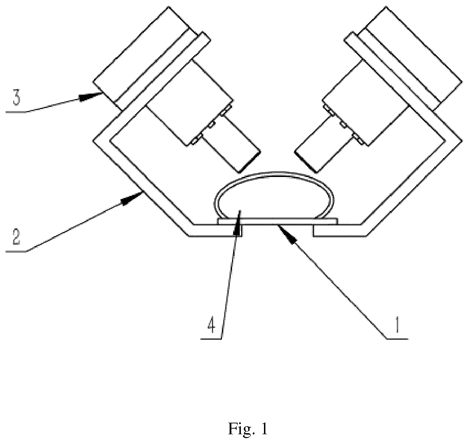

The present disclosure provides an ultrasonic control device. As shown in Fig. 1, the ultrasonic control device includes a fixed platform 1, movable supports 2, and ultrasonic transmitters 3. Here, the fixed platform 1 and the movable support are connected, for example, by a slide groove. The ultrasonic transmitter 3 may be fixed to the movable support 2 by screws. The fixed platform 1 is configured for placing an object to which ultrasonic waves are to be applied. Further, the human body or experimental animal 4 may be placed on the fixed platform 1 in the treatment or scientific research work for the human body or living animal. Furthermore, the ultrasonic control device includes a controller for controlling the frequency, energy and vibration direction of the ultrasonic waves emitted by the ultrasonic transmitter 3. Further, the ultrasonic transmitter 3 may be, for example, two, namely, a first ultrasonic transmitter and a second ultrasonic transmitter. Accordingly, the movable support 2 is, for example, two, namely, a first movable support and a second movable support. The first movable support and the second movable support are, for example, symmetrically and slidably connected to both sides of the fixed platform 1, respectively. The first ultrasonic transmitter is fixed to the first movable support and the second ultrasonic transmitter is fixed to the second movable support. However, it should be clear to a person skilled in the art that the number of ultrasonic transmitters 3 is not limited to two and may be at least one, e.g. one, two or three etc. When one ultrasonic transmitter is used, it is possible to adopt only one movable support and to face the ultrasonic transmitter to the human body or the experimental animal 4 so that the ultrasonic wave emitted by the ultrasonic transmitter is focused at the human body or the experimental animal 4.

Further, when the number of the ultrasonic transmitters is more than one, the different ultrasonic transmitters may, for example, be disposed at an angle so that the ultrasonic waves emitted by the different ultrasonic transmitters may intersect at the human body or the experimental animal 4. For example, the first ultrasonic transmitter and the second ultrasonic transmitter may be disposed perpendicularly and orthogonally to each other, so that the ultrasonic waves emitted by the first ultrasonic transmitter and the second ultrasonic transmitter may be perpendicularly and orthogonally disposed at the human body or the experimental animal 4.

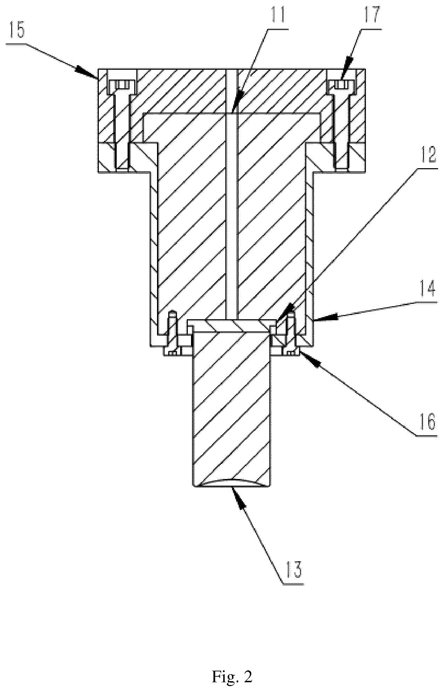

As shown in Fig. 2, the ultrasonic transmitter may include a base 11, a piezoelectric ceramic plate 12, an ultrasonic concentrator 13, a housing 14, an end cap 15, a front-end fixing screw 16, and a back-end fixing screw 17. The piezoelectric ceramic plate 12 may, for example, be sandwiched between the ultrasonic concentrator 13 and the base 11, and connected to the ultrasonic concentrator 13 and the base 11, for example, by a strong glue. The housing 14 may, for example, be fixedly connected to the end cap 15 to enclose the base 11 and the piezoelectric ceramic plate 12. The base 11 may be fixedly connected to the housing 14, e.g., by the front-end fixing screw 16. The end cap 15 may be fixedly connected to the housing 14, e.g., by the back-end fixing screw 17, and may be pressed onto the base 11, for example. The base 11 and the end cap 15 may, for example, be centrally aligned, and may each be provided with cable holes aligned up and down in a central region for routing cables.

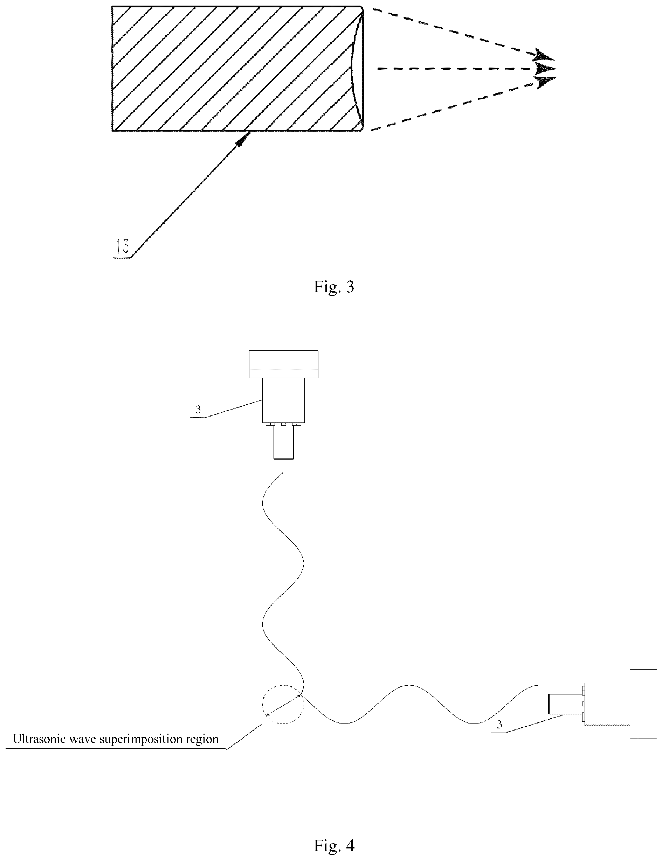

As shown in Fig. 3, the front end of the ultrasonic concentrator 13 is designed with an arc-shaped indent surface. When the piezoelectric ceramic plate 12 generates ultrasonic vibration, the ultrasonic vibration is transmitted to a front end having an arc-shaped indent surface, which focuses the generated ultrasonic wave at a certain distance, for example, 10 cm, from the front end using the arc-shaped indent surface, thereby concentrating the energy of the ultrasonic wave at a certain distance from the front end. It would have readily occurred to a person skilled in the art to focus the generated ultrasonic waves at different distances from the front end by setting the arc of the arc-shaped indent surface of the front end. It would have also readily occurred to a person skilled in the art that when more than one ultrasonic concentrator 13 is used, the arc of the arc-shaped indent surface of the front end of each ultrasonic concentrator may be the same or may be different, so that the ultrasonic waves generated by each ultrasonic concentrator may be focused at different distances from the end of the respective ultrasonic concentrator as required. Thus, it would have readily occurred to a person skilled in the art to achieve different focusing distances and focusing intensities of ultrasonic waves by different arc-shaped indent surface designs.

When the number of ultrasonic transmitters is more than one, the ultrasonic waves emitted by a plurality of ultrasonic transmitters may be superimposed, for example, at a certain region, preferably at a focusing region. The ultrasonic wave superimposition region is located, for example, at the human body or experimental animal 4. As shown in Fig. 4, two ultrasonic transmitters, such as a first ultrasonic transmitter and a second ultrasonic transmitter, are in an orthogonal arrangement, perpendicular to each other. By controlling the phases of the two high-frequency alternating voltages by the controller, the phases of the first ultrasonic transmitter and the second ultrasonic transmitter may be controlled, and the ultrasonic vibration superposition may be generated at the ultrasonic wave focusing position, thereby controlling the vibration direction of the superimposed ultrasonic waves. By controlling the amplitude of the high-frequency alternating voltage of the two paths of ultrasonic waves by the controller, it is possible to control the amplitude of the acoustic vibration, thereby controlling the energy at the focus of the ultrasonic waves, and thus controlling the ultrasonic energy at the ultrasonic wave superposition region.

Although Fig. 4 only shows the superposition phenomenon of ultrasonic waves generated by two ultrasonic transmitters (e.g. a first ultrasonic transmitter and a second ultrasonic transmitter), it would readily occur to a person skilled in the art that the number of ultrasonic transmitters may be different from two. For example, three ultrasonic transmitters, for example, in a triangular position, may be arranged above the human body or the experimental animal, and both set a focusing region somewhere on the human body or the experimental animal. Thus, three paths of ultrasonic waves emitted by the three ultrasonic transmitters are superimposed at the place of the human body or the experimental animal. Further, the three ultrasonic transmitters, for example, in an equilateral triangular position, may be arranged, above the human body or experimental animal.

The present disclosure also provides an ultrasonic control system including an ultrasonic control device as described above, and further including a deformable structure, which can be placed in a human or animal body, and which is made of an ultrasonic-responsive material and capable of releasing residual stresses under ultrasound excitation to cause deformation thereof.

In the treatment or scientific research work, the human body or the experimental animal implanted with a deformable structure may be placed on the fixed platform 1. The ultrasonic-responsive material of the implanted deformable structure is within the focus range of the ultrasonic concentrator of the ultrasonic transmitter. According to the frequency and energy required by the ultrasonic-responsive material, the frequency and amplitude of the output voltage of the controller are set, and then the high-frequency alternating voltage is started to be output. For example, when two ultrasonic transmitters are used, two paths of high-frequency alternating voltages are respectively output to the two ultrasonic transmitters. During one implementation period, the phase difference of the two paths of high-frequency alternating voltages gradually increases from 0 to 180 degrees, and then decreases to 0, so as to realize the omnidirectional scanning of ultrasonic vibration, which is beneficial to improve the driving capability of the ultrasonic-responsive material. Furthermore, the time and voltage amplitude of the on-period may be set during use, so as to realize slow long-time low-energy driving or short-time high-energy driving of the ultrasonic-responsive material, and realize the driving effect optimized for different ultrasonic-responsive materials. Among them, the ultrasonic-responsive polymer material is, such as polystyrene, polylactide and acrylonitrile based composite material.

Thus, the present disclosure achieves controlled compression of blood vessels or nerves within the human body or experimental animal by the ultrasonic transmitter and the ultrasonic-responsive material. The number, structure design and arrangement of ultrasonic transmitters are set to realize the focusing and superposition of ultrasonic waves at a specific region in the human body or experimental animal body. The frequency and amplitude of the output voltage of the controller are set to output high-frequency alternating voltage to realize the omnidirectional scanning of ultrasonic vibration, so as to improve the driving capability of the ultrasonic-responsive material, thereby optimizing the driving effect of the ultrasonic-responsive material and improving the control capability of the controlled compression of blood vessels or nerves in the human body or experimental animal body. Parts of the present disclosure that are not set forth in detail are well known in the art. The embodiments described above are merely illustrative of preferred implementations of the present disclosure. The preferred embodiments are not intended to be exhaustive and to describe all the details, or to limit the invention to the specific implementations described. Various changes and modifications to the disclosed embodiments can be made by those skilled in the art without departing from the spirit of the present disclosure, and it is intended that such changes and modifications fall within the scope of the appended claims.

## Claims

1. An ultrasonic control device, **characterized in that** the ultrasonic control device comprises:
a fixed platform configured for placing an object to which ultrasonic waves are to be applied;
a movable support slidably connected to the fixed platform;
an ultrasonic transmitter fixed to the movable support and configured for applying the ultrasonic waves to the object placed on the fixed platform; and
a controller configured for controlling the frequency, energy and vibration direction of the ultrasonic waves emitted by the ultrasonic transmitter.

2. The ultrasonic control device according to claim 1, **characterized in that** the movable support comprises a first movable support and a second movable support which are symmetrically and slidably connected to both sides of the fixed platform, respectively.

3. The ultrasonic control device according to claim 2, **characterized in that** the ultrasonic transmitter comprises a first ultrasonic transmitter fixed to the first movable support and a second ultrasonic transmitter fixed to the second movable support.

4. The ultrasonic control device according to claim 3, **characterized in that** the first ultrasonic transmitter and the second ultrasonic transmitter are disposed orthogonally to each other.

5. The ultrasonic control device according to claim 1, **characterized in that** the ultrasonic transmitter comprises a base, a piezoelectric ceramic plate, an ultrasonic concentrator, a housing and an end cap;
wherein the piezoelectric ceramic plate is sandwiched between the base and the ultrasonic concentrator; and
the housing is fixedly connected to the end cap to enclose the base and the piezoelectric ceramic plate.

6. The ultrasonic control device according to claim 5, **characterized by** further comprising a cable hole disposed in a central region of the end cap and the base.

7. The ultrasonic control device according to claim 5, **characterized in that** the ultrasonic concentrator has a front end which is an arc-shaped indent surface for focusing the energy of the ultrasonic waves.

8. The ultrasonic control device according to claim 1, **characterized in that** the movable support is connected to the fixed platform via a slide groove.

9. An ultrasonic control testing system, **characterized in that** the ultrasonic control testing system comprises the ultrasonic control device according to any of claims 1-8.

10. The ultrasonic control testing system according to claim 9, **characterized by** further comprising a deformable structure disposed within the object to which the ultrasonic waves are to be applied; and/or the deformable structure comprising an ultrasonic-responsive material.
